# EUROPEAN PATENT APPLICATION

(11) **EP 3 381 909 A1**
(43) Date of publication of application: **03.10.2018**
(21) Application number: 17305361.2
(22) Date of filing: 29.03.2017
(51) Int. Cl.: C07D 251/18, A61K 31/53, A61P 35/00

(54) **NEW TRIAZINE SUBSTANCE FOR USE IN THE TREATMENT OF CANCER, SPECIFICALLY IN THE TREATMENT OF NON-SMALL CELL CANCER, AND PHARMACEUTICAL COMPOSITION COMPRISING SAID SUBSTANCE**

(71) Applicant: Universite de Nantes, 44000 Nantes (FR)
(72) Inventor: ROUSSAKIS, Christos, 44100 NANTES (FR); ROBERT, Jean-Michel, 44200 NANTES (FR)
(74) Representative: Le Cloirec, Claudine

(57) **Abstract**

Substance of formula (I) for use as a medicament 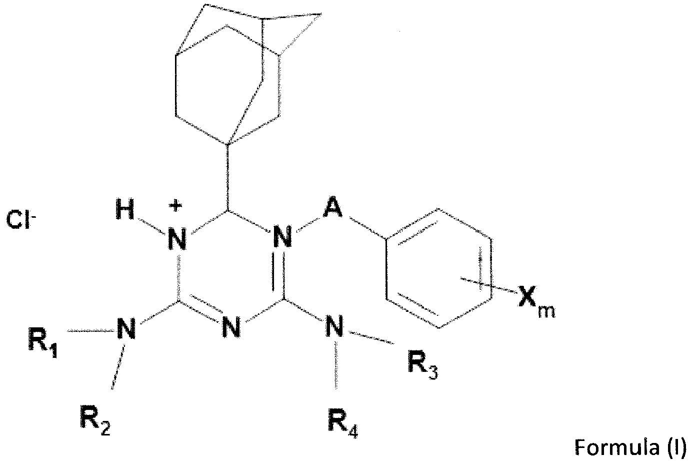 wherein A is a [-(CH₂)n-] spacer, n being an integer comprised between 1 and 3, preferably between 1 and 2 ; Xₘ are m substituents, m being an integer comprised between 1 and 3, wherein X is chosen among Cl, Br, OCH₃ or SCH₃; and R₁, R₂, R₃ and R₄ are chosen among hydrogen atoms or C₁-C₃ alkyl groups. This substance has an anti-proliferative action on tumor cells and may advantageously be for use in the treatment of non-small cell cancer, preferably for use in the treatment of non-small cell squamous carcinoma, and more preferably for use in the treatment of non-small cell lung cancer.

SEQUENCE LISTING

<110>  UNIVERSITE DE NANTES

<120>  New triazine substance for use in the treatment of cancer,
       specifically in the treatment of non-small cell cancer

<130>  PN000856

<160>  14

<170>  PatentIn version 3.5

<210>  1
<211>  20
<212>  DNA
<213>  artificial sequence

<220>
<223>  forward primer for gene FAK

<400>  1
tgacagggag gatggaagtc                                                   20


<210>  2
<211>  20
<212>  DNA
<213>  artificial sequence

<220>
<223>  reverse primer for gene FAK

<400>  2
tggagctgca ggatttcttt                                                   20


<210>  3
<211>  20
<212>  DNA
<213>  artificial sequence

<220>
<223>  forward primer for gene P130Cas

<400>  3
aaccccactg acaagaccag                                                   20


<210>  4
<211>  20
<212>  DNA
<213>  artificial sequence

<220>
<223>  reverse primer for gene P130Cas

<400>  4
tcccctgtag gtggacgtag                                                   20


<210>  5
<211>  20

<212>  DNA
<213>  artificial sequence

<220>
<223>  forward primer for gene HEF1

<400>  5
ccgctgccga aatgaagtat                                                    20


<210>  6
<211>  22
<212>  DNA
<213>  artificial sequence

<220>
<223>  reverse primer for gene HEF1

<400>  6
ccctgtgttc tgctctatga cg                                                 22


<210>  7
<211>  20
<212>  DNA
<213>  artificial sequence

<220>
<223>  forward primer for gene C3G

<400>  7
gacatcccag agatccctca                                                    20


<210>  8
<211>  20
<212>  DNA
<213>  artificial sequence

<220>
<223>  reverse primer for gene C3G

<400>  8
gacagctcgt ccacttcctc                                                    20


<210>  9
<211>  20
<212>  DNA
<213>  artificial sequence

<220>
<223>  forward primer for gene P13K

<400>  9
ccccgagcgt ttctgctttg                                                    20


<210>  10
<211>  19
<212>  DNA
<213>  artificial sequence

<220>
<223>  reverse primer for gene P13K

<400>  10
caagtggatg ccccacagt                                                    19


<210>  11
<211>  20
<212>  DNA
<213>  artificial sequence

<220>
<223>  forward primer for gene AKT

<400>  11
caggatgtgg accaacgtga                                                   20


<210>  12
<211>  20
<212>  DNA
<213>  artificial sequence

<220>
<223>  reverse primer for gene AKT

<400>  12
aaggtgcgtt cgatgacagt                                                   20


<210>  13
<211>  20
<212>  DNA
<213>  artificial sequence

<220>
<223>  forward primer for human gene beta-actin

<400>  13
attcccttgc cttcttggat                                                   20


<210>  14
<211>  20
<212>  DNA
<213>  artificial sequence

<220>
<223>  reverse primer for human gene beta-actin

<400>  14
aaggtgcgtt cgatgacagt                                                   20

## Description

### FIELD OF THE INVENTION

The present invention relates to the field of cancer treatment. More particularly the invention relates to a new substance for use as a medicament in the treatment of cancer, specifically for use in the treatment of non-small cell cancer.

### PRIOR ART

Cancer is still a serious public health problem. Although regular progresses are made in chimiotherapy, some types of cancer show little remission: this is the case of lung cancer, which is a major cause of morbidity and mortality worldwide: up to about 13 % of all cancer diagnoses in Europe and in the USA, with an overall 5-year survival rate which is only 15 %.

Non-small cell lung cancer (NSCLC) is found in about 85% of patients with lung cancer. Despite our growing insight into carcinogenesis, the treatment of tumors, especially non-small cell lung cancer (NSCLC), remains limited. The chemoresistance induced by current molecules (conventional or combination therapy) even in the early stages leads to many premature deaths and places the lung cancer in the first place of cancer deaths. It is therefore urgent to develop strategies that target tumor cells and their genetic features.

A synthetic triazine called A190 has already been disclosed by the inventors for use in the treatment of non-small cell cancer (PCT/GR02/00036). This substance has an anti-proliferative potential by a cytostatic activity that blocks cells in the G1 phase and induces apoptosis. In particular, it has been shown (Rousseau et al., Sci. Rep. 5, 10556, 2015) that said triazine A190 not only restores mutant p53 activity, but also induces an overexpression of the *NEDD9* gene, leading to apoptotic death.
The inventors have now discovered that a new molecule is surprisingly more efficient than the triazine A190 to treat such cancer tumors, at lower concentrations.

### SUMMARY OF THE INVENTION

The present invention therefore relates to a substance of formula (I) for use as a medicament 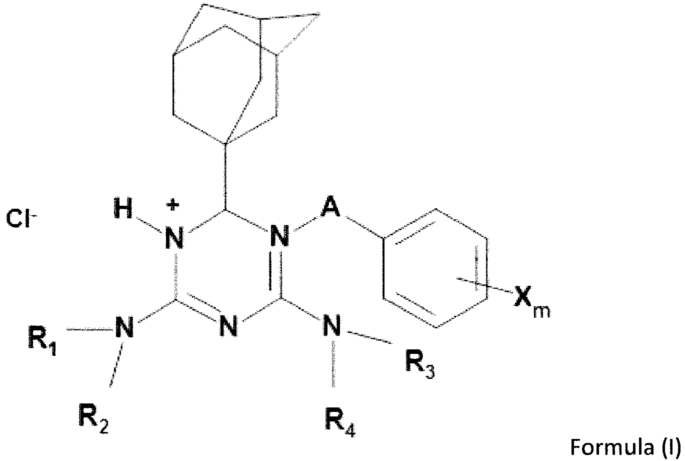
wherein A is a [-(CH₂)n-] spacer, n being an integer comprised between 1 and 3, preferably between 1 and 2 ;
Xₘ are m substituents, m being an integer comprised between 1 and 3, wherein X is chosen among Cl, Br, OCH₃ or SCH₃;
and R₁, R₂, R₃ and R₄ are chosen among hydrogen atom or C₁-C₃ alkyl groups.

In a preferred embodiment of the invention, A is -CH₂- in formula (I).

In formula (I) when m = 1, the substituent X₁ may be chosen among the following substituents:
a) X₁ = 3-, 4-, or 5-Cl; or
b) X₁ = 3- or 4-Br; or
c) X₁= 4-SCH₃; or
d) X₁ = 3- or 4- or 5-OCH₃;

When m = 2 in formula (I), the substituents X₁ and X₂ may be chosen among the following substituents:
e) X₁ = 3- or 4-Cl and X₂ = 5-Cl ; or
   3
f) X₁ = 3- or 4-OCH₃ and X₂ = 5-OCH₃; or
g) X₁ = 3-Br and X₂ = 5-Br.

When m = 3 in formula (I), the substituents X₁, X₂ and X₃ may be methoxy groups, respectively X₁= 3-OCH₃, X₂ = 4-OCH₃ and X₃ = 5-OCH₃.

In another preferred embodiment of the invention, R₁, R₂, R₃ and R₄ are all hydrogen atoms.

In still another preferred embodiment of the invention, R₁, R₃ and R₄ may be hydrogen atoms and R₂ a -CH₃ group.

In still another preferred embodiment of the invention, R₁ and R₃ may be hydrogen atoms and both R₂ and R₄ -CH₃ groups.

In still another preferred embodiment of the invention, R₃ is a hydrogen atom and R₁, R₂ and R₄ are -CH₃ groups.

More specifically the present invention relates to a substance of formula (II) as follows:

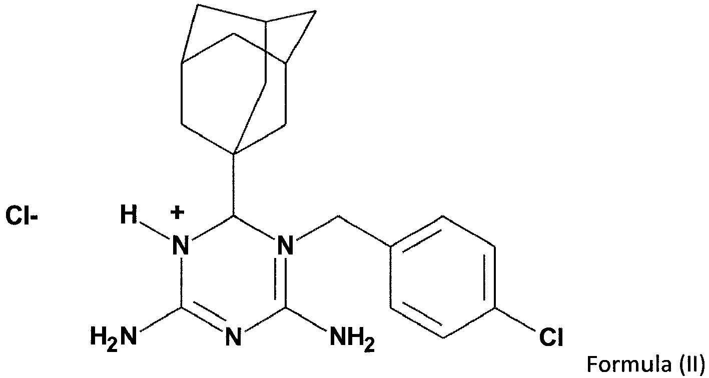

The substance as defined in formula (I) and (II) has been surprisingly shown to have an anti-proliferative action on tumor cells. Therefore, it may be for use in the prevention and/or the treatment of tumor cells proliferation.
According to the invention, the substance as defined above in Formula (I) or (II) may advantageously be is for use in the treatment of cancer, for instance for use in the treatment of non-small cell cancer, and preferably for use in the treatment of non-small cell squamous carcinoma.

More specifically, the substance of the present invention may be for use in the treatment of non-small cell lung cancer.

The present invention also relates to a pharmaceutical composition comprising at least one substance as defined above or a pharmaceutically acceptable salt thereof, as active pharmaceutical ingredient and at least one pharmaceutically acceptable ingredient, for use in the treatment of non-small cell lung cancer.

By "pharmaceutically acceptable ingredient" is meant any ingredient of pharmaceutical quality, that helps delivery, stability or biodisponibility of the active pharmaceutical ingredient and that can be metabolized and is non toxic to the patient.

Preferred pharmaceutically acceptable ingredients are chosen among commonly used excipients such as microcrystalline cellulose , lactose, starch or soy powder.

The pharmaceutically acceptable ingredient should be present in the pharmaceutical composition is an amount sufficient to have an inhibitory action on the target (tumor cells) and then an inhibitory action on tumor cells proliferation. For instance, the pharmaceutical composition of the present invention comprises, in weight %, from 0.01 % to 10 %, preferably from 0.02 % to 5 %, and more preferably from 0.05 % to 1 % of the total weight of the composition.

Said composition may be adapted to oral administration or intravenous injection. A therapeutically effective dose to be administrated to the patient may vary between about 10 mg/day and 1000 mg/day, preferably between 10 mg/day and 100 mg/day.

### DESCRIPTION OF THE FIGURES

The present invention is illustrated by the examples hereafter in relation with the annexed figures, in which:
Figure 1 is a graph showing the effect of comparative molecule A190 on cell growth of NSCLC-N6-L16 cell line in function of time;
Figure 2 is a graph showing the effect of comparative molecule A190 on cell growth of A549 cell line in function of time;
Figure 3 is a graph showing the effect of molecule of the invention A204 on cell growth of NSCLC-N6-L16 cell line in function of time;
Figure 4 is a graph showing the effect of molecule of the invention A204 on cell growth of A549 cell line in function of time;
Figure 5 is a histogram showing the expression of gene P53 in NSCLC-N6-L16 cells synchronised in M phase non-treated (control) or treated by 39.4µM of the molecule A204 of the present invention;
Figure 6 is a histogram showing the expression of gene HEF1 in NSCLC-N6-L16 cells synchronised in M phase non-treated (control) or treated by 39.4µM of the molecule A204 of the present invention;
Figure 7 is a histogram showing the expression of gene FAK in NSCLC-N6-L16 cells synchronised in M phase non-treated (control) or treated by 39.4µM of the molecule A204 of the present invention;
Figure 8 is a histogram showing the expression of gene C3G in NSCLC-N6-L16 cells synchronised in M phase non-treated (control) or treated by 39.4µM of the molecule A204 of the present invention;
Figure 9 is a histogram showing the expression of gene Pi3K in A549 cells synchronised in M phase non-treated (control) or treated by 39.4µM of the molecule A204 of the present invention;
Figure 10 is a histogram showing the expression of gene AKT in A549 cells synchronised in M phase non-treated (control) or treated by 39.4µM of the molecule A204 of the present invention;

### EXAMPLES

### Example 1: Synthesis of molecule of formula (II)

The full name of this molecule of formula (II) is: 4,6-diamino-1,2-dihydro-1-(4"-chlorobenzyl)-2-(1-tricyclo [3.3.1.1,4]decyl)-1,3,5-triazine (as hydrochloride salt); synonym : 4,6-diamino-1,2-dihydro-1-(4"-chlorobenzyl)-2-adamantyl-1,3,5-triazine.

### Step 1: synthesis of adamantyl carboxaldehyde (scheme 1).

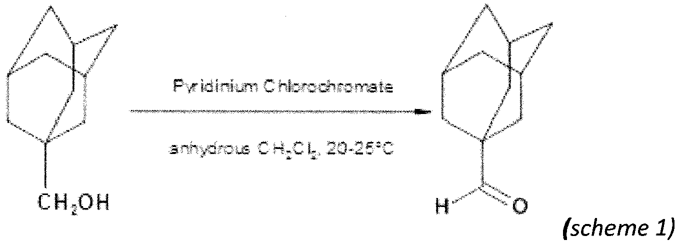

To a suspension of 3 g of pyridinium chlorochromate in 20 mL of anhydrous dichloromethane at 20-25°C, was added a solution of 1 g of adamantylmethanol in dry ethanol. The mixture was stirred at room temperature over 90 min and then was filtered under neutral silica. The filtrate was collected and the solvent removed under vacuum. The crude aldehyde was obtained and used at step 2 without further purification.

### Step 2: synthesis of the expected triazine, as hydrochloride salt (scheme 2).

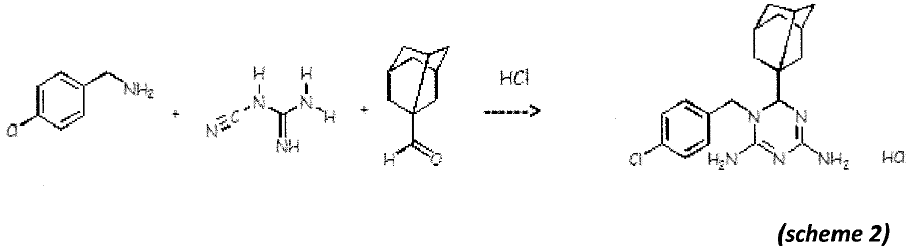

To a solution of 2.5 mM of (4-chlorophenyl)methylamine were added 2.5 mM of concentrated hydrochloric acid, 2.5 mM of cyanoguanidine and 2.77 mM of the aldehyde obtained at step 1. The expected triazine was obtained as a precipitate which was filtered, washed with absolute ethanol and dried.

### Example 2:

Two different substances were tested in the following examples.
- As comparative example another triazine derivative molecule named A190:

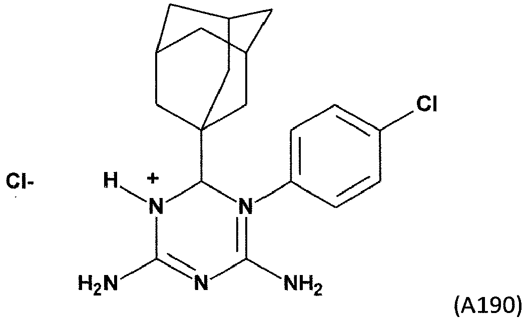

This substance A190 (4,6-diamino-1,2-dihydro-1-(400-chloro-phenyl)-2-(1-tricyclo[3.3.1.13,4] decyl-1,3,5-triazine) is a synthetic molecule which has already been described in the following publication: Rousseau et al., Sci. Rep. 5, 10556 (2015) and in the international patent application PCT/GR02/00036.
- As example of the present invention, the molecule of formula (II) also named A204 in the following examples is provided.

Both substances were dry and can be directly dissolved in water.

### Cell lines

A549 and NSCLC-N6 cell lines were used for all experiments. They were obtained respectively from an adenocarcinoma and an epidermoid lung cancer. The NSCLC-N6-L16 cell line was derived from a human non-small-cell bronchopulmonary carcinoma (moderately differentiated, classified as T2N0M0). The A549 was obtained from the ATCC collection (reference CCL-185) and is known to have a wild type p53 gene, while NSCLC-N6 has a mutant p53 gene, similar to the *in situ* tumors.

Both cell lines were cultured in RPMI-1640 medium with 5% fetal bovine serum, to which were added 100 IU penicillin/ml, 100 µg/mL streptomycin and 2 mM glutamine at 37°C in an air/carbon dioxide (95:5, v/v) atmosphere. The NSCLC-N6-L16 cell line is a model which never exceeded 35 passages in all experiments. Then this model stays very close to the original tumor.

### Cell synchronization in M phase

It was chosen to use the M phase synchronization instead of working with all the synchronization methods using biochemical agents as these latter methods can cause genotypic cell modifications that lead to bias.

During the mitosis, cell shape and adhesion are radically changed. Indeed, this phase induces a depolymerization of the microtubules, a loss of the cytoskeleton and consequently a decrease of the adhesion to the support. Hence, the cells are just lightly hanging at the bottom of the culture flask throughout the mitosis phase. It is thus easy to isolate these cells by a slight agitation of the culture medium in order to remove and suspend the cells. The cells are then recovered by slow centrifugation and replaced in a new culture flask.

### Cytotoxicity determinations: Continuous drug exposure

Experiments were performed in 96-well microtiter plates (10⁵ cells/mL for NSCLC-N6-L16 and 2X10⁴ cells/mL for A549). Cell growth was estimated by a colorimetric assay based on the conservation of tetrazolium dye (MTT) to a blue formazan product by live mitochondria. Eight repeats were performed for each concentration. Control growth was estimated from 8 determinations. Optical density at 570 nm corresponding to solubilized formazan was read for each well on a Titertek Multiskan MKII.

### Extraction of total RNA

Total RNA was extracted from NSCLC-N6-L16 and A549 cell lines using the Dynabeads^{®} mRNA DirectTM Kit (Ambion by Life Technologies). The isolation protocol relies on base pairing between the polyA residues at the 3' end of most mRNA and the oligo (dT) residues covalently coupled to the surface of the Dynabeads^{®}. The quality and concentration of purified RNA were assessed using UV absorbance at 260/280 nm and samples were run on 1% agarose gel in order to assess their quality. RNA was stored at - 80 °C.

### cDNA synthesis

RT was carried out with 1 µg of RNA in 10 µL with 0.125 µg/µL random primers and RNase-free water. This mixture was denatured at 65°C for 10 min then kept on ice. Subsequently, it was completed to a final volume of 25 µL with a mix containing 8 mM of dNTP mix, 1X of M- MLV 5X reaction buffer, 25 units of recombinant RNasin ribonuclease inhibitor (Promega), and 200 units of M-MLV RT (Promega) and RNase-free water. In order to check whether the samples were contaminated by genomic DNA, the same mix was made with RNA without the reverse transcriptase. Following this step, samples were incubated for 2 h at 37°C. cDNAs were stored at -20°C. After reverse transcription, cDNAs were used as a template for specific qPCR.

### Quantitative real-time RT-PCR

Real-time RT-PCR was carried out in a final volume of 25 µL with 1µL 1:20 dilution of diluted cDNA mixture, 10 pmol gene-specific forward and 10 pmol reverse primer in 1X SYBR Green PCR master mix (Eurogentec) with the following protocol: 15 s at 95 °C for denaturation, 30 s at 60°C for annealing and extension on a ABI Prism Sequence Detection System 5700 (Applied Biosystems). The primers were designed using Primer 3 (http://frodo.wi.mit.edu/). All primer sequences were controlled with a dimers finder to reduce the risk of unspecific hybridization of Sybr-green (Operon oligo toolkit: see https://www.operon.com(oligos/toolkit.php).

The primers used for all the experiments are listed in Table 1 below.

**Table 1**

| **Gene** | **Primer** | **Sequence (5' to 3')** | | **Tm* (°C)** |
|---|---|---|---|---|
| **FAK** | Forward | SEQ ID NO: 1 | TGACAGGGAGGATGGAAGTC | 64.2 |
| | Reverse | SEQ ID NO: 2 | TGGAGCTGCAGGATTTCTTT | 63.8 |
| **P130Cas** | Forward | SEQ ID NO: 3 | AACCCCACTGACAAGACCAG | 64.0 |
| | Reverse | SEQ ID NO: 4 | TCCCCTGTAGGTGGACGTAG | 63.9 |
| **HEF1** | Forward | SEQ ID NO: 5 | CCGCTGCCGAAATGAAGTAT | 56 |
| | Reverse | SEQ ID NO: 6 | CCCTGTGTTCTGCTCTATGACG | 68 |
| **C3G** | Forward | SEQ ID NO: 7 | GACATCCCAGAGATCCCTCA | 64.2 |
| | Reverse | SEQ ID NO: 8 | GACAGCTCGTCCACTTCCTC | 64 |
| **PI3K** | Forward | SEQ ID NO: 9 | CCCCGAGCGTTTCTGCTTTG | 64 |
| | Reverse | SEQ ID NO: 10 | CAAGTGGATGCCCCACAGT | 60 |
| **AKT** | Forward | SEQ ID NO: 11 | CAGGATGTGGACCAACGTGA | 62 |
| | Reverse | SEQ ID NO: 12 | AAGGTGCGTTCGATGACAGT | 60 |

| | | | | |
|---|---|---|---|---|
| *Tm: temperature | | | | |

The relative expression of each gene was normalized to that of human β-actin: forward 5'-ATTCCCTTGCCTTCTTGGAT-3' (SEQ ID NO: 13),
reverse 5'-CGTGAGGTCTGCCACTACAG-3' (SEQ ID NO: 14) (10 mM, Sigma Genosys). Normalization was carried out using the ΔΔCt method. Non-template control sample was used for each PCR to check the genomic DNA contaminations of the cDNA template. Analysis of the results was carried out using GenAmp 5700 SDS (Applied Biosystems) software.

### Results

The following results were obtained for substance A204, and were compared to the prior art substance A190:
***IC50:*** Half maximal inhibitory concentration
The concentration of both studied triazine substances A190 and A204 to inhibit activity of respectively NSCLC-N6-L16 and A549 are presented in table 2 below:

**Table 2**

| | **NSCLC-N6-L16** | **A549** |
|---|---|---|
| **A190 (µM)** | 24.07 ± 2.2 | 71.04 ± 7.2 |
| **A204 (µM)** | 5.01 ± 0.5 | 9.7 ± 0.7 |

It is observed that the IC₅₀ of A204 on NSCLC-N6-L16 is five times higher than that of A190, with 5.01 ± 0.5 µM vs 24.07 ± 2.2 µM.

The IC₅₀ of A549 on A549 is seven times higher than that of A190 with 9.7 ± 0.7 µM against 71.04 ± 1.2 µM.

The above results already show the superiority of the substance of the present invention over the prior art triazine A190.

It is therefore possible to proceed in a second step to the continuous growth kinetics.

### Example 3: Continuous drug exposure

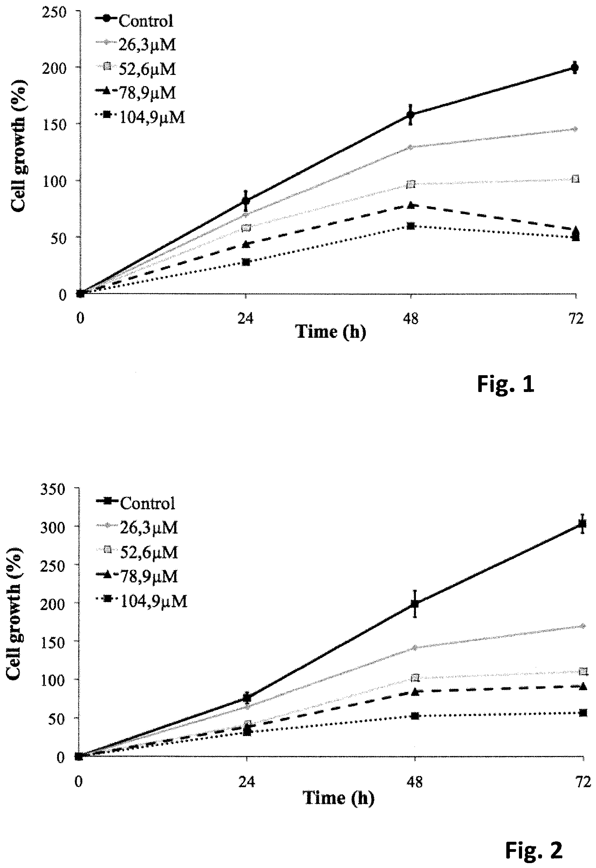

The kinetics were carried out on the two cell lines NSCLC-N6-L16 and A549 over a time of 72 h. The results are presented in the graphs of figures 1 to 4 representing the cell growth as a function of time. One of the curves represents the cell growth control; the others correspond to the different concentrations tested. The latter are chosen according to the IC₅₀ in order to surround it at best (Table 3).

**Table 3**

| **Substance / target** | | **Concentrations tested (µM)** | | | |
|---|---|---|---|---|---|
| **A190** | **L-16** | 26.3 | 52.6 | 78.9 | 104.9 |
| | **A549** | | | | |
| **A204** | **L-16** | 1.4 | 2.8 | 5.6 | 8.4 |
| | **A549** | 2.8 | 5.6 | 11.1 | 16.7 |

The two graphs of figures 1 and 2 show growth kinetics versus time after continuous exposure to drug at different concentrations. It can be noted that the proliferation is reduced for both cell lines in a dose-dependent manner without any toxicity at the highest doses. The formation of a "plateau" starts after 48 hours of treatment indicating the cell growth stopping without excessive mortality. A majority of cells are phase-dependent blocked and then cannot return in the cell cycle. These results show that the effect of the comparative substance A190 is irreversible and confirm its cytostatic activity against NSCLC-N6-L16 and A549 cell lines since 26.3 µM.

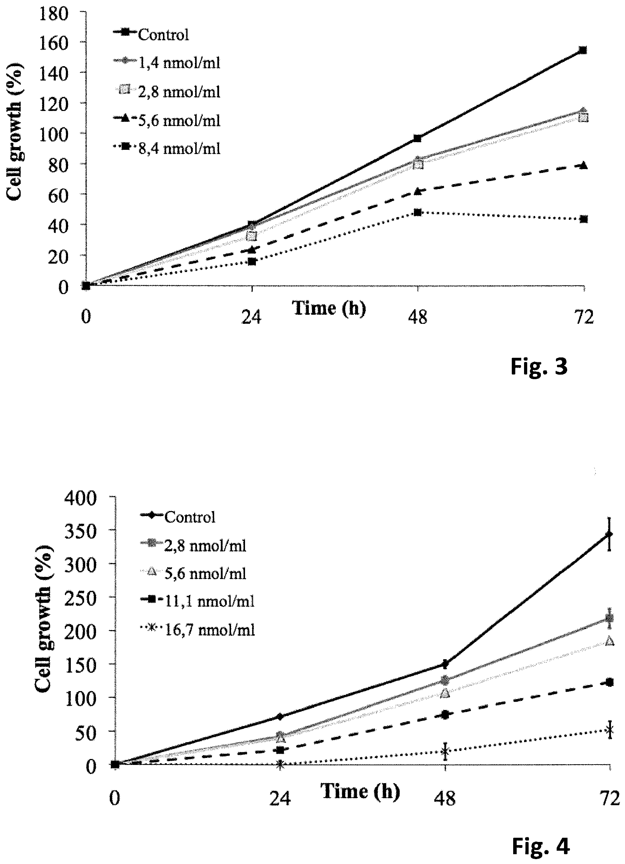

The profile observed in the growth of the NSCLC-N6-L16 line after treatment with the two molecules A190 and A204 is similar (see graphs of figures 1 and 3, respectively). Indeed, a « plateau » of cytostatic type is visible after 48 hours for the highest doses of A190: 104.9 µM, 78.9 µM and of A204: 8.4 µM, 5.6 µM.

The profile observed in the A549 cell line growth after treatment with the two molecules A190 and A204 is not similar (see graphs of figures 2 and 4, respectively). Indeed, a plateau of cytostatic type is visible after 48h of treatment with A190 for all the highest doses 104.9 µM, 78.9 µM and the weakest 52.6 µM, 26 µM. On the contrary, the kinetics of A549 cells treated with A204 follow perfectly that of the control cells, which is characteristic of an anti-proliferative profile.

### Example 4: Gene expression

The HEF1 gene is a homologue of the gene p130cas that is present during the G1 phase. During this phase, the proliferation pathways used by the cell (cancerous or not) begin mainly by an activation of the FAK protein that mainly passes through the integrins. It has been shown by previous studies that NSCLC-N6-L16 cell line, was following the pathway HEF1/C3G (in place of p130Cas) and A549 was following the pathway PI3K/AKT. Consequently, the researches have gone in those two directions.

### NSCLC-N6-L16

The expression of four genes of interests were studied in the cell line NSCLC-N6-L16: HEF1, p53, C3G and FAK. It was carried out by RT-QPCR on the synchronized cells not treated (dark histograms) or treated (grey histograms) by A240 at the IC₅₀. The study was performed between 10 and 17 hours in order to follow the gene expression with an extraction every hour.

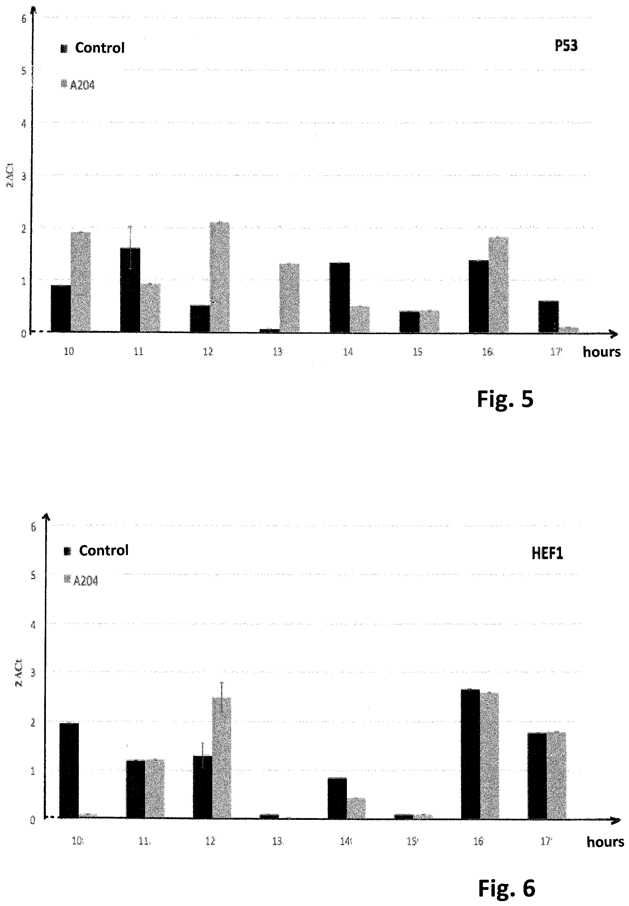

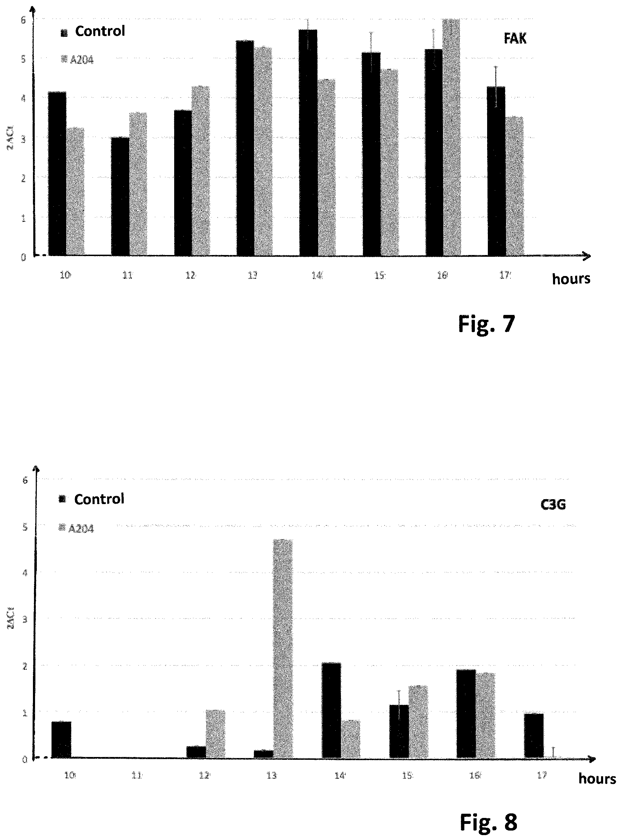

The results are presented on respective figures 5, 6, 7 and 8.

### A549

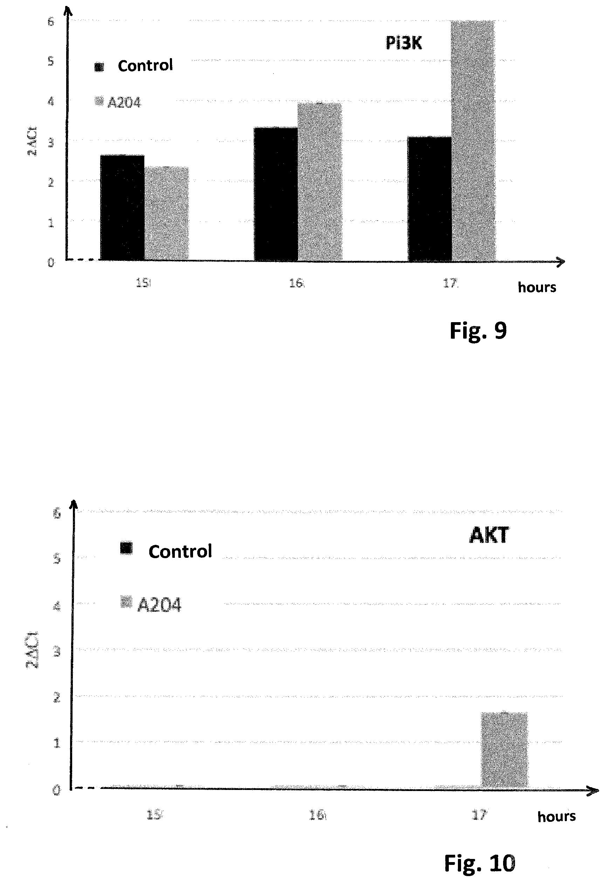

The expression of genes of interests PI3K and AKT were studied in the cell line A549. It was carried out by RT-QPCR on synchronized cells not treated (dark histograms) or treated (grey histograms) by A204 at the IC₅₀ (see figures 9 and 10).

### Molecular targets induction

The molecular analyses were performed on synchronized cells to facilitate the underline of the treatment effect on gene regulation.

It was observed that the cell doubling time of the cell line NSCLC-N6-L16 is about 17 hours and 22-23 hours for A549. Indeed, the molecule A204 act on different genes such as HEF1, the expression of which is variable along the cell cycle. Activity kinetics performed on synchronized cells shows that the effect of the treatment is beginning around 30 hours and after this point all cells seem to be blocked (data not shown). The comparison of these results to the kinetics done on normal cells confirms the real synchronization of the cells. Then the study conducted on the total RNA by quantitative RT-PCR, clearly shows an influence of the treatment on the expression of our molecular target HEF1, FAK, P53 and C3G for NSCLC-N6-L16 cell line and PI3K and AKT for A549 cell line.

The NSCLC-N6-L16 cells are synchronized in mitosis; the G1 phase for these cells is between 10 and 12 hours (Figures 5-8).
The **HEF1 gene** is highly overexpressed at around 12 hours for A204-treated cells. This overexpression blocks the cells in G1 phase and causes the induction of their apoptosis. Between 13 and 15 hours the expression level of HEF1 of the treated cells collapses; this corresponds to the phase of cell synthesis. Between 15 and 17 hours, this period corresponds to the G2 / M phase, the level of expression of HEF1 of the treated cells is relatively close to the level of the control cells. This corresponds to the cells which have escaped being blocked in phase G1.
Concerning the **FAK** study, the expression levels are relatively homogeneous except between 12 and 16 hours, which corresponds to the phase of synthesis. During this phase, we can observe an increased expression of the control cells and a decreased expression of the A204-treated cells. The synthesis phase of the cell cycle is accelerated when FAK is overexpressed. A decrease in its expression within the treated cells is therefore a sign that the A204 molecule acts on different targets at several levels of the cell cycle.
The gene **C3G** is widely overexpressed between 11 and 13 hours: this corresponds to the end of the G1 phase. This overexpression thus causes blocking of the cells treated in the G1 phase and the induction of apoptosis. Between 13 and 16h, the expression of C3G goes up gradually to finally collapse at 17 hours (period corresponding to phase G2 / M). These results demonstrate the effectiveness of the A204 molecule and the interest of targeting the C3G protein which blocks cells in the G1 phase and avoids their escapement in the G2 / M phase.

Previous work on the A190 line treated with A190 showed an overexpression of the **AKT and PI3K genes,** two oncogenes that lead to apoptosis after 17 hours of treatment. The same results are obtained for the treatment with the molecule A204 (Figures 9 and 10) ; an overexpression was observed also at 17 hours. The fact that this time the overexpression of the target genes of the A549 line is observed at the same time for the two molecules: A190 and A204, contrary to the targets studied on the NSCLC-N6-L16 line. This can be explained by the difference between the two cell lines doubling time.

### CONCLUSION

The new molecule of the present invention, A204, therefore acts just as A190. However, surprisingly, for the same cytostatic effect on NSCLC-N6-L16, A204 requires four times less amount and acts three hours earlier than A190 by infecting a signalling pathway (p53/ HEF1/ C3G) for epidermoid carcinomas. On A549, this molecule A204 requires seven times less product over a comparable action time but with an antiproliferative activity by infecting a more conventional signalling pathway (AKT, PI3K).

The above experiment has therefore shown the role and efficiency of the new molecule of the invention on non-small cell lung carcinoma tumors.

## Claims

**1.** Substance of formula (I) for use as a medicament 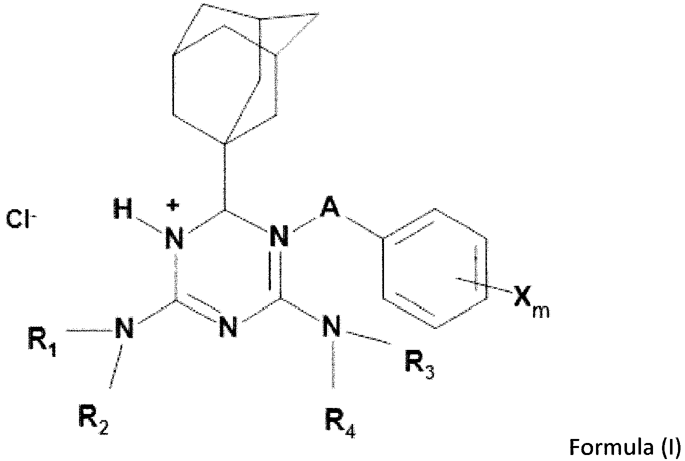
wherein A is a [-(CH₂)n-] spacer, n being an integer comprised between 1 and 3, preferably between 1 and 2 ;
Xₘ are m substituents, m being an integer comprised between 1 and 3, wherein X is chosen among Cl, Br, OCH₃ or SCH₃;
and R₁, R₂, R₃ and R₄ are chosen among hydrogen atoms or C₁-C₃ alkyl groups.

**2.** Substance according to claim 1 wherein A is -CH₂-.

**3.** Substance according to claim 1 or 2, wherein m = 1 and the substituent X₁ of formula (I) is chosen among the following substituents:
h) X₁ = 3-, 4-, or 5-Cl; or
i) X₁ = 3- or 4-Br; or
j) X₁ = 4-SCH₃; or
k) X₁ = 3- or 4- or 5-OCH₃;

**4.** Substance according to claim 1 or 2, wherein m = 2 and the substituents X₁ and X₂ of formula (I) are chosen among the following substituents:
I) X₁ = 3- or 4-Cl and X₂ = 5-Cl; or
m) X₁ = 3- or 4-OCH₃ and X₂ = 5-OCH₃; or
n) X₁ = 3-Br and X₂ = 5-Br.

**5.** Substance according to claim 1 or 2, wherein m = 3 and the substituents X₁, X₂ and X₃ of formula (I) are respectively X₁= 3-OCH₃, X₂ = 4-OCH₃ and X₃ = 5-OCH₃.

**6.** Substance according to any of the preceding claims, wherein R₁, R₂, R₃ and R₄ are all hydrogen atoms.

**7.** Substance according to any of claims 1 to 5, wherein R₁, R₃ and R₄ are hydrogen atoms and R₂ is a -CH₃ group.

**8.** Substance according to any of claims 1 to 5, wherein R₁ and R₃ are hydrogen atoms and R₂ and R₄ are -CH₃ groups.

**9.** Substance according to any of claims 1 to 5, wherein R₃ is a hydrogen atom and R₁, R₂ and R₄ are -CH₃ groups.

**10.** Substance according to anyone of claims 1, 2, 3 and 6, being of formula (II) as follows:

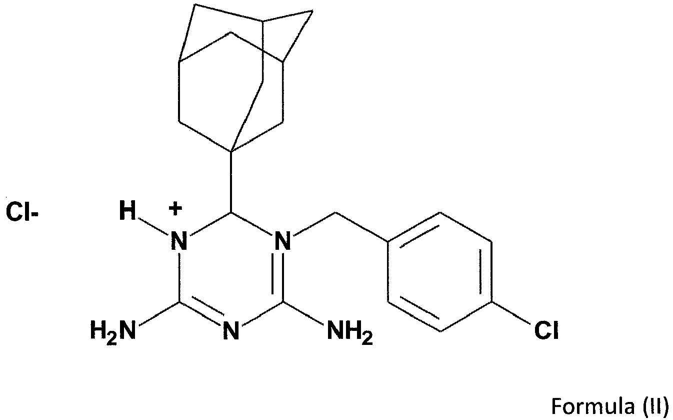

**11.** Substance according to anyone of the preceding claims, for use in the prevention and/or the treatment of tumor cells proliferation.

**12.** Substance according to anyone of the preceding claims, for use in the treatment of cancer.

**12.** Substance according to any of claims 11 or 12, for use in the treatment of non-small cell cancer, preferably for use in the treatment of non-small cell squamous carcinoma.

**14.** Substance according to anyone of claims 11 to 13 for use in the treatment of non-small cell lung cancer.

**15.** Pharmaceutical composition comprising at least one substance according to anyone of the preceding claims or a pharmaceutically acceptable salt thereof as active pharmaceutical ingredient, and at least one pharmaceutically acceptable ingredient, for use in the treatment of non-small cell lung cancer.
